# EUROPÄISCHE PATENTSCHRIFT

(11) **EP 3 706 833 B1**
(45) Veröffentlichungstag und Bekanntmachung des Hinweises auf die Patenterteilung: **16.08.2023**
(21) Anmeldenummer: 18822132.9
(22) Anmeldetag: 09.11.2018
(51) Int. Cl.: A61M 5/30, A61M 5/24, A61M 5/315

(54) **VERFAHREN UND VORRICHTUNG ZUM NADELLOSEN INJIZIEREN VON FLÜSSIGKEIT IN EIN SUBSTRAT SOWIE FLÜSSIGKEITSBEHÄLTNIS ZUR VERWENDUNG IN DEM VERFAHREN UND DER VORRICHTUNG**
METHOD AND DEVICE FOR THE NEEDLE-FREE INJECTING OF FLUID INTO A SUBSTRATE, AND FLUID CONTAINER FOR USE IN THE METHOD AND THE DEVICE
PROCÉDÉ ET DISPOSITIF D'INJECTION SANS AIGUILLE D'UN LIQUIDE DANS UN SUBSTRAT ET RÉCIPIENT DE LIQUIDE DESTINÉ À ÊTRE UTILISÉ DANS LE PROCÉDÉ ET LE DISPOSITIF

(30) Priorität: 10.11.2017 DE 102017126493
(43) Veröffentlichungstag der Anmeldung: 16.09.2020
(73) Patentinhaber: Conzima GmbH, 87487 Wiggensbach (DE)
(72) Erfinder: SCHMITT, Fritz, 6832 Betzdorf (LU)
(74) Vertreter: Angerhausen, Christoph
(86) Internationale Anmeldenummer: PCT/IB2018/058812
(87) Internationale Veröffentlichungsnummer: WO 2019/092643

(56) Entgegenhaltungen:
- WO-A1-2015/059707
- WO-A1-2016/102407
- US-A1- 2008 009 788
- US-A1- 2010 016 827

## Beschreibung

Die Erfindung ist auf eine Injektionsvorrichtung gemäß dem Oberbegriff des Anspruchs 1 zum nadellosen Injizieren von Flüssigkeit in ein Substrat gerichtet, insbesondere eines flüssigen pharmazeutischen oder kosmetischen Präparats in ein biologisches Gewebe, mit einem Flüssigkeitsvorrat, einer Auslassdüse und mit einer Flüssigkeit aus dem Vorrat durch die Auslassdüse in Form eines Flüssigkeitsstrahls ejizierenden Ej ektoreinrichtung.

Um eine Flüssigkeit in ein Substrat zu injizieren, beispielsweise ein flüssiges pharmazeutisches oder kosmetisches Präparat in oder unter die Haut eines Menschen oder anderen Lebewesens, wird die Flüssigkeit regelmäßig durch eine Injektionsnadel in das Substrat, also das menschliche oder tierische Gewebe eingeleitet. Die Injektionsnadel muss hierzu zunächst in das Substrat eingestochen werden. Infolge des dabei mittels einer Schneide an der Nadelspitze erzeugten Einschnitts kommt es zu Verletzungen, die in lebendem Gewebe zwar meist schnell wieder verheilen, aber regelmäßig eine Narbenbildung zur Folge haben. Außerdem bergen Injektionen mit Injektionsnadeln immer das Risiko einer Infektion.

Es hat daher in der Vergangenheit verschiedene Versuche mit hypodermischen Strahlinjektionsvorrichtungen für die nadellose Injektion gegeben, um unter Verzicht der Verwendung einer in das Substrat einstechbaren Injektionsnadel eine kleine Menge Flüssigkeit wie z.B. einen Impfstoff oder ein anderes Arzneimittel, ein
Anästhetikum oder dergleichen direkt durch die Hautoberfläche in das Gewebe einzubringen. Grundsätzlich bestand die Idee bei diesen Anstrengungen darin, die Haut des Patienten alleine durch den Druck der Flüssigkeit zu durchdringen und das Injektionsmedium in eine gewünschte Tiefe zu bringen. Die zu diesem Zweck entwickelten Geräte konnten allerdings die in sie gesetzten Erwartungen nicht erfüllen.

Die in der Vergangenheit zur nadellosen Injektion von Flüssigkeiten wie beispielsweise Arzneimitteln vorgeschlagenen Injektionsgeräte haben einen Energiespeicher wie beispielsweise einen Federspeicher, einen Druckspeicher und/oder eine Zündkapsel, der/die beim Auslösen eine Druckerhöhung in einem im Gerät enthaltenen Flüssigkeitsvorrat bewirkt, um aus dem Vorrat Flüssigkeit durch eine Auslassdüse auszustoßen. Der Düsenquerschnitt ist dabei möglichst klein und der auf den Flüssigkeitsvorrat wirkende Druck möglichst hoch, um einen Flüssigkeitsstrahl mit geringem Querschnitt und großer Strahlgeschwindigkeit zu erzeugen.

Aus der US 2002/0143323 A1 ist eine endoskopische Einrichtung zur gastrointestinalen Epithel-Entfernung bekannt, bei der eine Sonde mit einer Flüssigkeit versorgt wird. Die Flüssigkeit wird der Sonde aus einem Vorratsbehälter zugeführt, der mit unter Druck stehendem Gas aus einer Gasflasche beaufschlagbar ist. Die US 2006/0149193 A1 offenbart eine Einrichtung mit einer Sonde und einem Flüssigkeitsapplikator, der eine Flüssigkeitsaustrittsöffnung zur nadellosen Injektion einer Flüssigkeit in ein biologisches Gewebe und eine zu der Flüssigkeitsaustrittsöffnung führende Flüssigkeitsleitung aufweist. Eine zugeordnete Flüssigkeitsliefereinrichtung weist eine Antriebseinrichtung auf und ist mit einem druckspeichernden Druckbehälter als Energiespeicher verbindbar. Zu der Flüssigkeitsliefereinrichtung gehört eine Expansionskammer, die eine bewegliche Wandfläche aufweist, die die zu injizierende Flüssigkeit umschließt und die mit einer Druckflüssigkeit beaufschlagbar ist.

Weiter sind Geräte bekannt, die zur nadellosen Injektion einer Flüssigkeit unter die Mucosa dienen. Zum Beispiel offenbart die US 2009/0157114 A1 ein Endoskop mit einer Sonde zur nadellosen Unterspritzung der Mucosa. Die Sonde stößt dazu einen Strahl Natriumchloridlösung aus, der aufgrund seines geringen Querschnitts und seiner gleichzeitig hohen Geschwindigkeit in das Gewebe eindringt. Um die Natriumchloridlösung zu fördern und den entsprechenden Druck zu erzeugen, ist eine Pumpeinheit oder gegebenenfalls ein kraftverstärkender Hebel vorgesehen.

Die bekannten Vorrichtungen haben sich bislang als wenig erfolgreich erwiesen, denn der damit erzeugte Flüssigkeitsstrahl geht schon unmittelbar nach seinem Austritt aus der Auslassdüse auseinander und verringert zugleich seine Geschwindigkeit. Beim Auftreffen auf das Substrat neigt er dann dazu "aufzupilzen" , also auseinander zu spritzen, so dass zumindest ein Teil der auf die Substratoberfläche gerichteten Flüssigkeit nicht in das Substrat eindringt, sondern seitlich der Aufprallstelle abläuft. Dies hat dann aber auch zur Folge, dass unklar ist, ob und welche Menge Flüssigkeit tatsächlich in das Substrat injiziert wurde. Mit den bekannten Vorrichtungen ist es insbesondere nicht möglich, Flüssigkeit in eine gewünschte Tiefe in dem Substrat einzubringen und in dieser Tiefe ein Flüssigkeitsdepot mit einer bestimmten Flüssigkeitsmenge anzulegen. weitere relevante Dokumente aus dem Stand der Technik sind US2010/016827A1, US2008/0009788A1, WO2015/059707A1, sowie WO2016/102407A1.

Aufgabe der Erfindung ist es daher, eine Vorrichtung der eingangs genannten Art zu schaffen, womit eine zuverlässige Injektion von Flüssigkeit in ein Substrat ohne Verwendung einer in das Substrat eingestochenen Injektionsnadel (Kanüle) möglich ist. Diese Aufgrabe wird durch eine Vorrichtung nach Anspruch 1 gelöst. Vorteilhafte Ausführungsformens sind Gegenstand der abhängigen Ansprüche.

Mit der Vorrichtung nach der Erfindung wird zunächst eine erste Teilmenge Flüssigkeit mit sehr hoher Geschwindigkeit als feiner Flüssigkeitsstrahl mit geringem Querschnitt aus der Auslassdüse ausgestoßen. Dazu ist es möglich, dass zumindest in der ersten Teilmenge Flüssigkeit ein Impulsstoß (Druckstoß) erzeugt wird, der bewirkt, dass diese erste Teilmenge infolge des plötzlich auf einen sehr hohen Wert ansteigenden Flüssigkeitsdrucks die Vorrichtung als Vorstrahl mit sehr großer Geschwindigkeit verlässt und auf das Substrat auftrifft und bei geeigneter Wahl einer an der Vorrichtung vorgesehenen Ejektionsdüse wegen des dann sehr geringen Strahldurchmessers ohne nennenswerten Widerstand in das Substrat bis in eine gewünschte Tiefe eindringt. Es ist auch möglich, die große Energie für die Erzeugung des feinen Flüssigkeitsstrahls mit hoher Austrittsgeschwindigkeit aus der Auslassdüse dadurch zu erzeugen, dass die Flüssigkeit in der Injektionsvorrichtung zunächst mit einem sie aufnehmenden Flüssigkeitsbehältnis auf eine Anfangsgeschwindigkeit beschleunigt und dann am Ende der Beschleunigungsstrecke zwar das Flüssigkeitsbehältnis an einem Anschlag abgebremst wird, die Flüssigkeit aber jedenfalls zu einem Teil ihre Bewegung durch die Auslassdüse hindurch fortsetzt. Bei dieser vorteilhaften Vorgehensweise wird der Flüssigkeit durch die Beschleunigung ind er Injektionsvorrichtung eine dynamische Druckkomponente aufgeprägt, entsprechend geringer fällt der statische Druckanstieg (Impulsstoß) in der Flüssigkeit aus, wenn das Flüssigkeitsbehältnis nach dem Beschleunigungsvorgang wieder abgebremst wird und die Flüssigkeit aus dem Behältnis durch die Auslassdüse ausgeschoben wird.

Die Eindringtiefe des die Auslassdüse verlassenden Flüssigkeitsstrahls in das Gewebe ist - neben seinem Strahldurchmesser - abhängig von der Initialgeschwindigkeit der Flüssigkeit (dynamische Druckkomponente), auf die diese in der Vorrichtung beschleunigt wird, und der Stärke des Impulses (statische Druckkomponente), mit dem die Flüssigkeit, insbesondere die erste Teilmenge, beaufschlagt wird. Bevorzugt ist die Flüssigkeit bzw. die erste Teilmenge in der Vorrichtung zunächst in einem Aufnahmeraum für den Flüssigkeitsvorrat aufgenommen und wird jedenfalls an einem Bereich von einem Stoßeinleitungselement zur Einleitung des Impulsstoßes begrenzt. An dem Stoßeinleitungselement, bei dem es sich ganz allgemein grundsätzlich um ein wie auch immer geartetes Mittel oder eine Ausgestaltung des Flüssigkeitsvorrats bzw. dessen Aufnahmeraums handeln kann, womit es ermöglicht ist, einen Druckstoß (Impulsstoß) in die im Aufnahmeraum aufgenommene Flüssigkeit einzuleiten, kann ein hierfür vorgesehenes Betätigungsmittel der Ejektoreinrichtung, das nach Auslösung der Einrichtung auf hohe Geschwindigkeit beschleunigt wird, also insbesondere ein Ejektorstößel, angreifen. Der dem Betätigungsmittel aufgrund seiner Masse und seiner hohen Geschwindigkeit innewohnende Stoßimpuls wird beim Anschlag des Betätigungsmittels am Stoßeinleitungselement des Flüssigkeitsvorrats/Aufnahmeraums, bzw. bei einer Ausführungsform, bei der dieser zusammen mit der darin aufgenommenen Flüssigkeit und dem Ejektorstößel gemeinsam auf eine Geschwindigkeit beschleunigt wird, beim Auftreffen des Flüssigkeitsvorrats an einem dafür vorgesehenen Anschlag im Gehäuse, in die in dem Aufnahmeraum aufgenommene Flüssigkeit übertragen und bewirkt in dieser einen plötzlichen, sehr großen Druckanstieg (Druckstoß), der zur Folge hat, dass eine erste Teilmenge der im Vorrat aufgenommenen Flüssigkeit mit entsprechend hohem Druck ausgestoßen wird. Wenn der Ausstoß durch eine Düse mit kleinem Querschnitt erfolgt, führt dies am Düsenauslass, wo der Flüssigkeit dann Umgebungsdruck aufgeprägt wird, zu einer sehr hohen Austrittsgeschwindigkeit des Flüssigkeitsstrahls der ausgestoßenen ersten Teilmenge. Die infolge des Druckstoßes austretende erste Teilmenge verlässt also die Auslassdüse entsprechend dem sehr hohen, kurzfristig in der Flüssigkeit erzeugten Druck mit sehr großer Geschwindigkeit als ein - in einer bevorzugten Ausgestaltung um seine Strahlachse rotierender - Flüssigkeitsstrahl und dringt ohne weiteren nennenswerten Widerstand in das Substrat ein. In dem Substrat erzeugt der Flüssigkeitsstrahl einen an der Substratoberfläche offenen Injektionskanal, der bis in eine Injektionstiefe reicht. Diese erreichte Tiefe hängt im Wesentlichen ab von der Strahlgeschwindigkeit, mit der die erste Teilmenge auf der Substratoberfläche auftrifft, sowie von der Strahldicke, die sich im Wesentlichen aus dem Querschnitt der Auslassdüse ergibt, durch die die Flüssigkeit die Vorrichtung verlässt. Die Geschwindigkeit des Strahls wiederum stellt (u.a.) eine Funktion des Drucks dar, der infolge des Impulsstoßes in der Flüssigkeit für tatsächlich nur sehr kurze Zeit erreicht wird. Indem man also die Geschwindigkeit des Betätigungsmittels (Ejektorstößel) und damit die Größe des diesem verliehenen Impulses variiert, kann man die Injektions- oder Eindringtiefe in das Substrat gezielt beeinflussen. Dabei hat sich überraschend gezeigt, dass auch eine zweite Teilmenge Flüssigkeit, die nachfolgend aus der Düse der Vorrichtung ausgestoßen wird, problemlos in den vorab mittels der ersten Teilmenge erzeugten Injektionskanal in das Substrat eindringt, und zwar selbst dann, wenn sie mit deutlich geringerem Druck durch die Auslassdüse ausgestoßen wird und dementsprechend mit geringer Geschwindigkeit an dem Substrat auftrifft. Diese zweite Teilmenge tritt dann ohne weiteren Widerstand an der Substratoberfläche in den zuvor erzeugten, an der Substratoberfläche offenen Injektionskanal ein, und zwar bis zu dessen Ende, also bis in die Eindringtiefe, in der sich die Flüssigkeit dann im Wesentlichen gleichmäßig um den Kanal herum verteilt. Eine zweite Teilmenge der Flüssigkeit kann also nach Art eines Flüssigkeitsdepots in die gewünschte Tiefe eingebracht werden.

Zur Durchführung einer derartigen, zwei- oder auch mehrstufigen Injektion hat sich als Ejektoreinrichtung eine solche mit elektromagnetischem Antrieb für den Ejektorstößel besonders bewährt. Der Ejektorstößel wird von dem elektromagnetischen Antrieb zunächst in einer Beschleunigungsstrecke vor dem Flüssigkeitsbehältnis (oder gemeinsam mit diesem) auf die gewünschte, hohe Stößelgeschwindigkeit beschleunigt und dann (ggf. zusammen mit dem Flüssigkeitsbehältnis) zur Erzeugung des Impulsstoßes in der Flüssigkeit binnen sehr kurzer Zeit, ggf.schlagartig abgebremst, woraufhin der Druck in der Flüssigkeit wie beschrieben plötzlich auf einen sehr hohen Wert ansteigt und die erste Teilmenge Flüssigkeit aus dem Behältnis ejiziert wird und die Auslassdüse mit sehr hoher Geschwindigkeit, bevorzugt unter Rotation, also einer schraubenförmigen Bewegung verlässt. Zum Injizieren einer zweiten (und möglichen weiteren) Teilmenge in den derart mittels der ersten Teilmenge erzeugten Injektionskanal wird mittels des elektromagnetischen Antriebs der Ejektorstößel nach Vorbild eines Spritzenkolbens einer Injektionsspritze mit einer Ejektionskraft am Stoßeinleitungselement in das Volumen der im Flüssigkeitsvorrat aufgenommenen Flüssigkeit eingeschoben und treibt diese dadurch durch die Auslassdüse aus, von wo aus sie in den zuvor mittels der ersten Teilmenge in das Substrat geschossenen Injektionskanal gelangt. Der Flüssigkeitsvorrat ist also bevorzugt mittels eines von der Ejektionseinrichtung betätigbaren Ejektionskolbens beaufschlagbar, welcher wiederum von dem Ejektionsstößel beaufschlagbar ist oder von diesem gebildet wird.

Der elektromagnetische Antrieb kann sich an einem von der Auslassdüse bzw. einem Anschlag für den Flüssigkeitsvorrat oder den Ejektorstößel beabstandeten, rückwärtigen Ende des Gehäuses oder etwa in dessen Mitte befinden, wobei sich die Beschleunigungsstrecke zwischen der Auslassdüse bzw. dem Anschlag und dem rückwärtigen Gehäuseende erstreckt.

Die Anordnung kann so getroffen sein, dass der elektromagnetische Antrieb eine am Ejektionsstößel selbst ausgebildete Magnetspule sowie einen den Ejektionsstößel umgebenden Eisenzylinder und/oder eine Ständerspule aufweist. Der Ejektionsstößel kann mit einer Stromspeichereinrichtung versehen sein, um den elektromagnetischen Antrieb mit Strom zu versorgen. Natürlich ist es auch möglich, eine externe Stromversorgung vorzusehen, um die Vorrichtung mit Energie zu versorgen.

Da die Beschleunigungsstrecke im Bereich vor und/oder hinter dem Ejektionsstößel mit Druckausgleichsöffnungen in Verbindung ist, wird ein Einfluss von Luftkompression bzw. eines entstehenden Unterdrucks in der Beschleunigungsstrecke auf die Bewegung des Ejektorstößels weitestgehend unterbunden. Es ist dabei möglich, dass die Druckausgleichsöffnungen über eine Überströmleitung miteinander verbunden sind, so dass die auf dem Weg des Ejektorstößels vor diesem befindliche Luft durch die Überströmleitung hinter den Stößel
strömen und somit für einen besonders zuverlässigen, schnellen Druckausgleich sorgen kann.

Wie bereits erwähnt, weist die Ejektionseinrichtung in vorteilhafter Ausgestaltung der Erfindung Mittel zum Erzeugen einer Druckerhöhung im Flüssigkeitsvorrat in unmittelbarem Anschluss an den ausgeübten Impulsstoß auf, die zweckmäßig im Wesentlichen von dem Ejektionsstößel gebildet werden, der nach Ausübung des Impulsstoßes mittels eines kraftausübenden Antriebs auf den Flüssigkeitsvorrat wirkt. Der kraftausübende Antrieb ist dabei bevorzugt der elektromagnetische Antrieb. Wenn der Flüssigkeitsvorrat in einem Flüssigkeitsbehältnis aufgenommen ist, das austauschbar im Gehäuse anordbar ist, kann die Auslassdüse am Flüssigkeitsbehältnis angeordnet sein, womit in jedem Fall sichergestellt ist, dass für eine bestimmte, zu injizierende Flüssigkeit auch die bestmöglich passende Auslassdüse zur Verwendung kommt.

Es hat sich überraschend gezeigt, dass ein Aufweiten, also eine Querschnittsvergrößerung, des Flüssigkeitsstrahls auf seinem Weg von der Injektionsvorrichtung zur Substratoberfläche und das bei den bekannten Vorrichtungen immer wieder beobachtete Aufpilzen beim Auftreffen auf das Substrat sehr zuverlässig vermieden wird, wenn der Flüssigkeitsstrahl bei seinem Auftreffen auf das Substrat mit bevorzugt hoher Strahlgeschwindigkeit und geringem Strahlquerschnitt um seine eigene Achse (Strahlachse) rotiert. Es wird angekommen, dass infolge der Rotation wirkende Zentripetalkräfte die Flüssigkeitsteilchen (Moleküle) zusammenhalten, und zwar nicht nur auf dem Weg des Flüssigkeitsstrahls von der Auslassdüse zur Substratoberfläche, sondern auch beim Eindringen in das Substrat. Tatsächlich scheint, jedenfalls bei geeigneter Gestaltung der Auslassdüse, es durch die Rotation des Strahls nach dessen Austritt aus der Auslassdüse sogar zu einer Querschnittsverringerung und damit zu einer Geschwindigkeitserhöhung des Flüssigkeitsstrahls zu kommen, so dass dieser auf das Substrat sogar mit einer größeren Geschwindigkeit auftreffen kann, als er beim Austritt aus einer Auslassdüse aufweist. Versuche haben gezeigt, dass der Flüssigkeitsstrahl bei einer Injektion in biologisches Gewebe wie z.B. in oder unter die Haut eines Menschen oder eines Tieres zuverlässig in dieses eindringt, auch wenn die Auslassdüse der erfindungsgemäßen Vorrichtung in einem Abstand von der Gewebeoberfläche positioniert ist, der Flüssigkeitsstrahl also die Distanz zwischen der Düse und der Gewebeoberfläche als "Freistrahl" zu überbrücken hat, und zwar ohne dass eine Vergrößerung der Distanz einen nachteiligen Einfluss auf die Injektionsqualität haben würde. Die Rotation, die dem Strahl vor seinem Auftreffen auf das Substrat aufgeprägt wird, überlagert sich mit der translatorischen Bewegung der Flüssigkeit in dessen Strahlrichtung zu einer schraubenförmigen Bewegung, mit der sich der Strahl nach den gemachten Beobachtungen mit sehr geringem Widerstand an der Oberfläche des Substrats in dieses unter Ausbildung eines dem Strahlquerschnitt entsprechenden Einlasskanals praktisch "hineinbohrt" oder "hineinschraubt", wobei tatsächlich praktisch keine der auf das Substrat auftreffenden Flüssigkeit verlorengeht, also nicht mit in das Substrat eindringt. Vorzugsweise wird wenigstens die erste Teilmenge Flüssigkeit vor und bei ihrem Durchgang durch die Auslassdüse der erfindungsgemäßen Vorrichtung in Rotation um ihre Strahlachse versetzt.

In vorteilhafter Ausgestaltung der Efindungkann die Rotation des Flüssigkeitsstrahls mittels mindestens einer Blende oder Düse mit mindestens einem schraubengang- oder wendelförmig verlaufenden Fluidkanal bewirkt werden. Dementsprechend kann die Injektionsvorrichtung nach der Erfindung als Mittel, um den Flüssigkeitsstrahl in Rotation zu versetzen, vorzugsweise mindestens einen etwa schraubengang- oder wendelförmigen Fluidkanal an der Auslassdüse umfassen. Mit Hilfe des mindestens einen schraubengang- oder wendelförmigen Fluidkanals wird zunächst wenigstens dem von der ersten Teilmenge gebildeten ersten Teilstrom der durch die Auslassdüse strömenden Flüssigkeit zumindest am Außenumfang des Flüssigkeitsstrahls, also im Grenzbereich zur umgebenden Luft, die erwünschte rotatorische Bewegung aufgeprägt, wobei sich diese Rotations- oder Schraubbewegung ins Innere des Flüssigkeitsstrahls überträgt. Es ist auch möglich, dass die Rotation des Flüssigkeitsstrahls mittels einer rotierenden Blende oder Düse bewirkt wird, wozu die vorrichtungsmäßig vorgesehenen Mittel vorzugsweise mindestens einen rotierend antreibbaren Teil der Auslassdüse umfassen. Auch eine Kombination der beiden rotationserzeugenden Maßnahmen ist denkbar. Es ist auch möglich, im Falle einer gemeinsamen Beschleunigung von Ejektorstößel und dem die Flüssigkeit enthaltenden Flüssigkeitsvorrat diese beiden über die Beschleunigungsstrecke in Längsrichtung bewegten Bestandteile der Vorrichtung zugleich um ihre Längsachse in eine Drehung zu versetzen, so dass die im Vorrat aufgenommene Flüssigkeit beim Auftrffen des Flüssigkeitsvorrats an dem Anschlag bereits verwirbelt/in Rotation versetzt ist und diesen Drehimpuls (Verwirbelung) beim Ausstoß durch die Auslassdüse beibehält.

Die erfindungsgemäße Injektionsvorrichtung kann bevorzugt so ausgestaltet sein, dass der Flüssigkeitsvorrat, die Auslassdüse und die Ejektoreinrichtung in einem gemeinsamen Gehäuse angeordnet/anordbar sind. Die Vorrichtung lässt sich auf diese Weise besonders kompakt gestalten, sie ist beispielsweise als Injektionsvorrichtung zum Injizieren von kosmetischen oder pharmazeutischen Flüssigkeiten in bzw. unter die Haut eines Menschen oder eines Tieres auch mit nur einer Hand problemlos handhabbar.

In vorteilhafter Weiterbildung der Injektionsvorrichtung kann der mindestens eine, schraubengang- oder wendelförmige Fluidkanal an einer einen Durchgang in der Auslassdüse begrenzenden Düsenwandung angeordnet sein. Die Anordnung kann bei der Injektionsvorrichtung derart getroffen sein, dass die Auslassdüse wenigstens einen konvergierenden Abschnitt aufweist, dessen Querschnitt sich in Durchströmrichtung der ejizierten Flüssigkeit verringert, so dass die Flüssigkeit bei ihrem Weg durch den konvergierenden Abschnitt der Auslassdüse beschleunigt wird. In diesem Fall hat es sich als vorteilhaft erwiesen, wenn der mindestens eine Fluidkanal sich zumindest über eine Teillänge des konvergierenden Abschnitts erstreckt.

Die Auslassdüse kann auch mindestens einen Abschnitt konstanten Querschnitts aufweisen, wobei sich der mindestens eine Fluidkanal dann bevorzugt (auch) zumindest über eine Teillänge des Abschnitts mit konstantem Querschnitt erstreckt.

Eine besonders effektive Maßnahme, um der durch die Auslassdüse strömenden Flüssigkeit die gewünschte Rotations- oder Schraubbewegung aufzuprägen, besteht in mehreren, im Wesentlichen rotationssymmetrisch zur Achse des Flüssigkeitsstrahls in der Auslassdüse angeordneten Fluidkanälen. Durch die Mehrzahl von Fluidkanälen erhält man eine vergleichsweise große, schrauben- bzw. wendelförmig verlaufende Kontakt- bzw. Austauschfläche zwischen dem Düsendurchlass und der durch diesen hindurchströmenden Flüssigkeit, womit ein starker Drall bzw. eine vergleichsweise schnelle Rotation der Flüssigkeit beim Düsenaustritt auch schon bei kurzer axialer Erstreckung der Düse (Düsenlänge) erreichbar ist. Die Fluidkanäle können benachbart zueinander an der den Durchlass der Auslassdüse begrenzenden Durchlasswandung angeordnet sein.

Eine ebenfalls besonders vorteilhafte Ausgestaltung besteht darin, dass der mindestens eine Fluidkanal sich in Form einer gewendelten Rohrleitung von der Einlass- zur Auslassseite der Auslassdüse durch diese erstreckt. Bei diese Ausführungsform wird die rotatorische Bewegungskomponente, die der Flüssigkeitsstrahl nach seinem Durchgang durch die Auslassdüse aufweist, durch die Wendelform der Rohrleitung bewirkt, durch die wenigstens ein Teilstrom Flüssigkeit strömt und infolge der unterschiedlichen Radien an der Innenseite und der Außenseite der Rohrwendel in Drehung um die Achse seines Stromfadens im Inneren der Leitung versetzt wird. Wenn die Rohrleitung zusätzlich einen sich von der Einlassseite zur Auslassseite hin abnehmenden Wendelradius aufweist, führt dies in außergewöhnlich vorteilhafter Weise zu einem Zykloneffekt, nämlich zu einer Erhöhung der Strömungsgeschwindigkeit des (rotierenden) Flüssigkeitsstrahls bei dessen Austritt aus der derartig gestalteten Auslassdüse. Man kann eine derart ausgebildete Düse also als Zyklondüse bezeichnen. Der beschriebene Effekt lässt sich weiter verstärken, indem zwei oder mehr gewendelte Rohrleitungen jeweils um einen Winkelbetrag zueinander versetzt nach Art einer Doppel- oder Mehrfachhelix vorgesehen werden. Den Effekt einer derartigen "Zyklondüse" kann man auch mit einem oder mehreren an der Durchlasswandung einer Düse mit einem sich verengenden, insbesondere konischen Düsendurchlass und an dessen Wandung angeordneten, wendelartig verlaufenden Fluidkanälen erreichen.

Eine ebenfalls zweckmäßige Gestaltung ist es, wenn die Auslassdüse einen zentralen, vorzugsweise gerade verlaufenden Durchlass für einen Teilstrom der Flüssigkeit aufweist und wenn der mindestens eine Fluidkanal den zentralen Durchlass schraubenförmig koaxial umgibt. Durch den den zentralen Durchlass insbesondere schraubenförmig umgebenden Fluidkanal strömt dann ein (zweiter) Teilstrom und erhält dabei wie vorstehend beschrieben eine Schraubenbewegung aufgeprägt, bevor er sich nach dem Austritt aus der Düse mit dem (ersten) Teilstrom vereinigt und seine Rotations- bzw. schraubenförmige Bewegung in diesen überträgt, so dass der aus beiden Teilströmen bestehende gesamte Flüssigkeitsstrom in erfindungsgemäß vorteilhafter weise um seine Strahlachse rotiert, während er aus der Düse auf das Substrat auftrifft und sich praktisch in dieses hineinschraubt oder -bohrt.

Wie bereits angedeutet, kann die Auslassdüse drehbar gelagert und mittels eines Antriebs in Rotation versetzbar sein. Dabei weist sie vorzugsweise mindestens einen, insbesondere vorzugsweise mehrere exzentrisch zur Achse des aus der Auslassdüse ejizierten Flüssigkeitsstrahls angeordnete(n) Fluidkanal/-kanäle auf. Die Drehbewegung der Auslassdüse bzw. des/der darin angeordnete(n) Fluidkanals/-kanäle um die Achse des Flüssigkeitsstrahls übertragen ihre Drehbewegung auf diesen, so dass der Flüssigkeitsstrahl bei seinem Austritt aus der Düse die erfindungsgemäße Rotationsbewegung um seine Strahlachse hat.

Eine insbesondere in produktionstechnischer Hinsicht besonders zweckmäßige Gestaltung ergibt sich, wenn die Auslassdüse mehrere in Durchlassrichtung der Flüssigkeit hintereinander in Form eines Blendenstapels angeordnete Blendenscheiben aufweist, die jeweils eine sich über einen Teil des Scheibendurchmessers erstreckende Schlitzöffnung aufweisen, wobei die Schlitzöffnungen von in dem Blendenstapel aufeinander folgenden Blendenscheiben in Umfangsrichtung um einen Winkelbetrag zueinander versetzt angeordnet sind. Die übereinander gestapelten Blendenscheiben mit den darin angeordneten, um einen Winkelbetrag versetzt ausgerichteten Schlitzöffnungen bilden dann einen zentralen, im Wesentlichen geradlinig in Achsrichtung der Düse verlaufenden Durchlass sowie zwei nach Art einer Doppelhelix angeordnete, wendeltreppenartig gestufte Fluidkanäle entlang der sich ausbildenden Wandung des zentralen Durchlasses. Dabei ist die Anordnung bevorzugt so getroffen, dass der Betrag des Versatzes in Umfangsrichtung an den radial äußeren Enden der Schlitzöffnungen kleiner ist als die Breite der Schlitzöffnungen, so dass die wendel-(treppen)förmige Wirkung der den zentralen Durchlass schraubengangförmig umgebenden Fluidkanäle bis in deren radial äußersten Randbereiche sichergestellt ist.

Es ist insbesondere für den Einsatz der Injektionsvorrichtung als kosmetisches und oder pharmazeutisches Gerät von besonderem Vorteil, wenn der Flüssigkeitsvorrat von einem Flüssigkeitsbehältnis gebildet wird, das bevorzugt austauschbar im Gehäuse angeordnet werden kann. Wenn die Flüssigkeit in einem austauschbar im Gehäuse aufgenommenen Flüssigkeitsbehältnis, beispielsweise in Form einer Kartusche oder einer Ampulle, aufgenommen ist, lassen sich mit ein und derselben Vorrichtung mit geringstmöglichen Aufwand nicht nur unterschiedliche Flüssigkeiten injizieren, also beispielsweise flüssige pharmazeutische Präparate unterschiedlicher Art, wie sie bei einer Impfserie benötigt werden können, indem einfach nacheinander Behältnisse mit verschiedenen Flüssigkeiten in die Vorrichtung eingesetzt werden. Die Anordnung hat darüber hinaus den Vorteil, dass sich die Vorrichtung ohne den darin aufgenommenen Flüssigkeitsvorrat besonders einfach und gründlich reinigen und/oder sterilisieren lässt, was insbesondere bei ihrem Einsatz in pharmazeutischen Bereichen, aber auch im (kommerziellen) kosmetischen Bereich von Bedeutung ist.

Als sehr vorteilhaft hat es sich erwiesen, wenn die Auslassdüse am Flüssigkeitsbehältnis angeordnet ist. Diese Anordnung erlaubt es in besonders einfacher Weise, die Art und Form der Düse, insbesondere den darin vorgesehenen Durchlass für die Flüssigkeit, bestmöglich an die spezifische, im Flüssigkeitsbehältnis aufgenommene und zu injizierende Flüssigkeit anzupassen. Beispielsweise kann es erforderlich sein, bei der Verarbeitung von Flüssigkeiten mit vergleichsweise hoher Viskosität wie beispielsweise Hyaluronsäurepräparaten, die in kosmetischen Anwendungsbereichen z.B. zur Faltenunterspritzung oder zum Modellieren von Lippen und in der Medizin zum Einspritzen in arthrosegeschädigte Gelenke zum Einsatz kommen, eine Düse mit größerem Durchlassquerschnitt vorzusehen als zum Einspritzen von einfacher, physiologischer Kochsalzlösung. Die Anordnung der Auslassdüse unmittelbar am Flüssigkeitsbehältnis stellt dann sicher, dass in jedem Fall die passende Auslassdüse für die jeweilige im Behältnis aufgenommene Flüssigkeit zum Einsatz kommt. Insbesondere aus hygienischen Gründen wird bevorzugt, dass es sich bei den in der erfindungsgemäßen Injektionsvorrichtung zum Einsatz kommenden Flüssigkeitsbehältnissen, insbesondere solchen mit daran angeordneten Auslassdüsen, um Einmal-Behältnisse handelt, die nach einmaliger Verwendung entsorgt, also nicht wieder befüllt werden.

Die Auslassdüse kann einen im Wesentlichen koaxial zur Gehäuseachse des Gehäuses verlaufenden Düsenauslass aufweisen. Die Flüssigkeit tritt dann in einer koaxial zur Gehäuseachse des Gehäuses verlaufenden Richtung aus und damit im Allgemeinen senkrecht auf die Oberfläche des Substrats auf, denn bei der Handhabung des Geräts wird das Gehäuse im Allgemeinen lotrecht zur Substratoberfläche, beispielsweise eine Hautfläche, ausgerichtet. Es ist aber in besonders vorteilhafter Weise auch möglich, dass die Auslassdüse einen Düsenauslass aufweist, der unter einem Winkel zur Gehäuseachse verläuft, wobei der Winkel vorzugsweise größer ist als 45°. Ganz besonders vorteilhaft ist es, wenn der Düsenauslass in einer Richtung liegt, die im Bereich von über 75° bis hin zu einem rechten Winkel oder sogar darüber hinaus liegt, die Auslassrichtung also im Wesentlichen in einer Normalebene zur Gehäuseachse des Gehäuses verläuft. Bei im Wesentlichen gleichbleibender, also etwa lotrecht zur Substratoberfläche gewählter Ausrichtung des Gehäuses ermöglicht es diese Ausgestaltung der Erfindung, die Flüssigkeit im Wesentlichen parallel zur Substratoberfläche dicht unter diese in das Substrat zu injizieren, was besonders einfach dann erreichbar ist, wenn das Substrat wie beispielsweise die Haut eines Menschen in seiner/ihrer oberen Schicht nachgiebig ist und mithilfe der Vorrichtung ein Stück weit muldenartig eingedrückt werden kann, so dass sich der Düsenauslass dann in dieser muldenartigen ausbildenden Vertiefung unterhalb des Niveaus des benachbarten Substrats befindet und dann die Flüssigkeit im Wesentlichen parallel zur Substratoberfläche unterhalb von dieser injiziert werden kann. Insbesondere für eine derartige Auslassdüse kann diese oder das vordere Ende des Gehäuses mit einer Tiefenanzeige oder einem Tiefenanschlag versehen sein, so dass die Flüssigkeit genau in die gewünschte Tiefe unterhalb der Substratoberfläche eingebracht werden kann.

In besonders vorteilhafter Ausgestaltung der Erfindung ist vorgesehen, dass das Flüssigkeitsbehältnis mit der darin aufgenommenen Flüssigkeit zusammen mit einem Ejektionsstößel der Ejektoreinrichtung in dem Gehäuse bzw. einer darin vorgesehenen Beschleunigungsstrecke beweglich aufgenommen ist und dass das Gehäuse an seinem vorderen Auslassende einen Anschlag für das Flüssigkeitsbehältnis aufweist. Diese Ausgestaltung hat den Vorteil, dass das Flüssigkeitsbehältnis zusammen mit der darin aufgenommenen Flüssigkeit zunächst gemeinsam mit der Ejektoreinrichtung in dem Gehäuse beschleunigt wird, bevor die Flüssigkeit aus ihrem Behältnis durch die Auslassdüse ausgestoßen wird. Hierdurch wird die Druckerhöhung (Erhöhung des statischen Druckes) in der Flüssigkeit bei Betätigung der Ejektoreinrichtung zum Ejizieren der Flüssigkeit begrenzt, indem der Flüssigkeit zunächst eine dynamische Druckkomponente aufgeprägt wird. Besonders bei druckempfindlichen Flüssigkeiten kann hierdurch die Gefahr einer Beschädigung verringert oder ganz vermieden werden. Um den (statischen) Druckanstieg in der Flüssigkeit beim Auftreffen des Flüssigkeitsbehältnisses an dem Anschlag zu verlangsamen, ist es vorteilhaft, wenn ein zwischen dem Anschlag und das Flüssigkeitsbehältnis wirksamer Anschlagdämpfer, beispielsweise ein Elastomer-Pufferelement vorgesehen ist.

Mit der Erfindung wird ein Flüssigkeitsbehältnis in der Vorrichtung nach der Erfindung vorgeschlagen, das gekennzeichnet ist durch mindestens einen Aufnahmeraum für Flüssigkeit, einen Flüssigkeitsauslass und ein Stoßeinleitungselement zur Einleitung eines Impulsstoßes in die im Aufnahmeraum aufgenommene Flüssigkeit. An dem Stoßeinleitungselement, bei dem es sich ganz allgemein grundsätzlich um ein wie auch immer geartetes Mittel oder eine Ausgestaltung des Flüssigkeitsbehältnisses handeln kann, womit es ermöglicht ist, einen Druckstoß (Impulsstoß) in die im Flüssigkeitsbehältnis aufgenommene Flüssigkeit einzuleiten, kann ein hierfür vorgesehenes Betätigungsmittel der Ejektoreinrichtung, das nach Auslösung der Einrichtung auf hohe Geschwindigkeit beschleunigt wird, also insbesondere der bereits erwähnte Ejektorstößel, angreifen. Der dem Betätigungsmittel aufgrund seiner Masse und seiner hohen Geschwindigkeit innewohnende Stoßimpuls wird beim Anschlag des Betätigungsmittels am Stoßeinleitungselement des Flüssigkeitsbehältnisses, bzw. bei der Ausführungsform, bei der dieses gemeinsam mit der darin aufgenommenen Flüssigkeit und dem Ejektorstößel gemeinsam auf eine Geschwindigkeit beschleunigt wird, beim Auftreffen des Flüssigkeitsbehältnisses an dem dafür vorgesehenen Anschlag im Gehäuse, in die in dem Behältnis aufgenommene Flüssigkeit übertragen und bewirkt in dieser einen plötzlichen, sehr großen Druckanstieg (Druckstoß), der zur Folge hat, dass eine erste Teilmenge der im Behältnis aufgenommenen Flüssigkeit mit entsprechend hohem Druck durch die Auslassdüse gepresst wird, wobei ihr beim Durchgang durch den Düsendurchlass bevorzugt eine Drehbewegung aufgeprägt wird. Die infolge des Druckstoßes austretende erste Teilmenge verlässt also die Auslassdüse entsprechend dem sehr großen, kurzfristig in der Flüssigkeit erzeugten Druck mit sehr großer Geschwindigkeit als ein (vorzugsweise) um seine Strahlachse rotierender Flüssigkeitsstrahl und dringt ohne weiteren nennenswerten Widerstand in das Substrat ein. In dem Substrat erzeugt der rotierende Flüssigkeitsstrahl einen an der Substratoberfläche offenen Injektionskanal, der bis in eine Injektionstiefe reicht. Diese erreichte Tiefe hängt im Wesentlichen ab von der Strahldicke, die sich im Wesentlichen aus dem Querschnitt der Auslassdüse ergibt, durch die die Flüssigkeit die Vorrichtung verlässt, und von der Strahlgeschwindigkeit, mit der die erste Teilmenge auf der Substratoberfläche auftrifft. Diese Geschwindigkeit wiederum stellt (u.a.) eine Funktion des Drucks dar, der infolge des Impulsstoßes in der Flüssigkeit für tatsächlich nur sehr kurze Zeit erreicht wird. Indem man also die Geschwindigkeit des Betätigungsmittels (Ejektorstößel) und damit die Größe des diesem verliehenen Impulses variiert, kann man die Injektions- oder Eindringtiefe in das Substrat gezielt beeinflussen. Dabei hat sich überraschend gezeigt, dass auch eine nachfolgend durch den vorab mittels der ersten Teilmenge erzeugten Injektionskanal in das Substrat eingebrachte zweite Teilmenge Flüssigkeit, die dann üblicherweise mit deutlich geringerem Druck durch die Auslassdüse ausgestoßen und dementsprechend mit geringer Geschwindigkeit in das Substrat injiziert wird, in diesem bis zum Ende des zuvor erzeugten Injektionskanals, also bis in die Eindringtiefe, gelangt und sich in dieser dann im Wesentlichen gleichmäßig um den Kanal herum verteilt. Eine zweite Teilmenge Flüssigkeit kann also nach Art eines Flüssigkeitsdepots in die gewünschte Tiefe eingebracht werden. Zur Durchführung einer derartigen, zwei- oder mehrstufigen Injektion hat sich als Ejektoreinrichtung eine solche mit elektromagnetischem Antrieb für den Ejektorstößel besonders bewährt. Der Ejektorstößel wird von dem elektromagnetischen Antrieb zunächst in einer Beschleunigungsstrecke vor dem Flüssigkeitsbehältnis (oder gemeinsam mit diesem) auf die gewünschte, hohe Stößelgeschwindigkeit beschleunigt und dann (ggf. zusammen mit dem Flüssigkeitsbehältnis) zur Erzeugung des Impulsstoßes in der Flüssigkeit in sehr kurzer Zeit, praktisch schlagartig abgebremst, woraufhin der Druck in der Flüssigkeit wie beschrieben plötzlich auf einen sehr hohen Wert ansteigt und die erste Teilmenge Flüssigkeit aus dem Behältnis ejiziert wird und die Auslassdüse mit sehr hoher Geschwindigkeit, bevorzugt unter Rotation, also einer schraubenförmigen Bewegung verlässt. Zum Ejizieren einer zweiten (und möglichen weiteren) Teilmenge(n) in den derart mittels der ersten Teilmenge erzeugten Injektionskanal wird mittels des elektromagnetischen Antriebs der Ejektorstößel nach Vorbild eines Spritzenkolbens einer Injektionsspritze mit einer Ejektionskraft am Stoßeinleitungselement in das Volumen der im Behältnis aufgenommenen Flüssigkeit eingeschoben und treibt diese dadurch durch die Auslassdüse aus, von wo aus sie in den zuvor mittels der ersten Teilmenge in das Substrat geschossenen Injektionskanal gelangt.

Die Ejektoreinrichtung mit elektromagnetischem Antrieb, der eine eigenständige erfinderische Leistung zukommt und die sich selbstverständlich auch für Verfahren und Vorrichtungen eignet, bei denen die zur nadellosen Injektion in die Form eines dünnen Strahls beschleunigte Flüssigkeit nicht in Rotation bzw. Schraubenbewegung versetzt wird, ermöglicht nicht nur die vorstehend beschriebene, gestufte Injektion mit zwei oder mehreren Flüssigkeitsteilmengen. Sie eignet sich auch in ganz vorzüglicher Weise dazu, eine Serie von Injektion in kurzer zeitlicher Abfolge an verschiedenen, bevorzugt unmittelbar zueinander benachbarten Stellen im Substrat zu platzieren. Hierzu wird der Ejektorstößel unmittelbar nach Erzeugen des Impulsstoßes in dem Flüssigkeitsvorrat, bevorzugt durch kurzzeitiges Umkehren der Stromrichtung in der Spule, wieder zurück in seine Ausgangsstellung verstellt und ist so binnen kürzester Zeit für einen weiteren Injektionsvorgang bereit, zu dem er mittels der elektromagnetischen Spule wieder in Injektionsrichtung beschleunigt wird und erneut einen Druckimpuls in dem Flüssigkeitsvorrat erzeugt. Besonders für die Durchführung solcher Serieninjektionen eignet sich ein einen Zylinderraum bildendes Flüssigkeitsbehältnis mit einem an einer Seite vorgesehenen Flüssigkeitsauslass und einem von dem Ejektorstößel betätigbaren Kolben, der in von dem auf ihn auftreffenden Stößel bewirkten Schritten in den Zylinderraum einschiebbar ist, um immer eine Teilmenge Flüssigkeit aus dem Flüssigkeitsauslass auszustoßen, die dann die Vorrichtung durch die Auslassdüse verlässt. Da der Kolben mit jedem Injektionsvorgang von dem Ejektorstößel zunehmend tief in den Zylinderraum des Flüssigkeitsbehältnisses eingeschoben wird, vergrößert sich schrittweise die Beschleunigungsstrecke, die dem Ejektorstößel zwischen einem hinteren, gleichbleibenden Anschlag im Gehäuse und seinem vorderen, vom Kolben definierten Anschlagpunkt zur Verfügung steht. Da die Vergrößerung der Beschleunigungsstrecke bei ansonsten unveränderten Rahmenbedingungen, insbesondere gleichbleibender Stromstärke, mit der der elektromagnetische Antrieb beaufschlagt wird, eine zunehmend größere Geschwindigkeit des Stößel bei seinem Auftreffen auf den Kolben zur Folge hätte, womit dann auch der in der Flüssigkeit erzeugte Druckimpuls und die daraus resultierende Strahlgeschwindigkeit und Eindringtiefe ansteigen, sind bevorzugt Mittel zur Anpassung der Stößelgeschwindigkeit bei dessen Auftreffen auf den Kolben vorgesehen, die es ermöglichen, unabhängig von der Stellung des Kolbens im Flüssigkeitsbehältnis in diesem wiederholbar zumindest annähernd gleich starke Druckimpulse zu erzeugen, so dass die in Serie erzeugten Injektionen jeweils bis in dieselbe Tiefe in das Substrat eindringen. Die erfindungsgemäße Vorrichtung eignet sich somit in vorteilhafter Weise für das Unterspritzen von Falten in der Haut eines Patienten oder aber auch zur Erzeugung von Tätowierungen, die mit der Erfindung nadellos erzeugt werden können.

Es ist auch möglich, dass sich der Elektromagnet und/oder der zu dessen Betrieb vorgesehene Energiespeicher (Batterie/Akku) an dem beweglichen Teil der Ejektoreinrichtung, also insbesondere dem Ejektorstößel befinden und gemeinsam mit diesem aus dem Gehäuse entfernt werden kann/können, bspw. um dieses vor einem erneuten Geräteeinsatz zu reinigen und/oder zu sterilisieren.

Weitere Merkmale und Vorteile der Erfindung ergeben sich aus der nachfolgenden Beschreibung und der Zeichnung, worin bevorzugte Ausführungsformen der Erfindung anhand von Beispielen dargestellt und näher erläutert sind. Es zeigt:
- Fig. 1: eine Übersichtsdarstellung einer erfindungsgemäßen Injektionsvorrichtung in perspektivischer Ansicht;
- Fig. 2a - c: das Handteil der Injektionsvorrichtung nach Fig. 1 im Längsschnitt in verschiedenen Arbeitsstellungen der Ejektoreinrichtung;
- Fig. 3: die Ejektoreinrichtung mit einer ersten Ausführungsform einer bei der Erfindung zum Einsatz kommenden Auslassdüse, im Längsschnitt;
- Fig. 4: eine zweite Ausführungsform einer Auslassdüse zum Einsatz mit der erfindungsgemäßen Vorrichtung im Schnitt;
- Fig.5: eine dritte Ausführungsform einer Auslassdüse zum Einsatz mit der erfindungsgemäßen Vorrichtung im Schnitt;
- Fig. 6: eine vierte Ausführungsform einer Auslassdüse zum Einsatz mit der erfindungsgemäßen Vorrichtung im Schnitt;
- Fig. 7: die Blendenscheiben des bei der Ausführungsform nach Fig. 6 zum Einsatz kommenden Blendenstapels in einer perspektivischen, auseinander gezogenen Darstellung (Explosionsdarstellung); und
- Fig. 8: eine fünfte Ausführungsform einer Auslassdüse zum Einsatz mit der erfindungsgemäßen Vorrichtung im Schnitt.

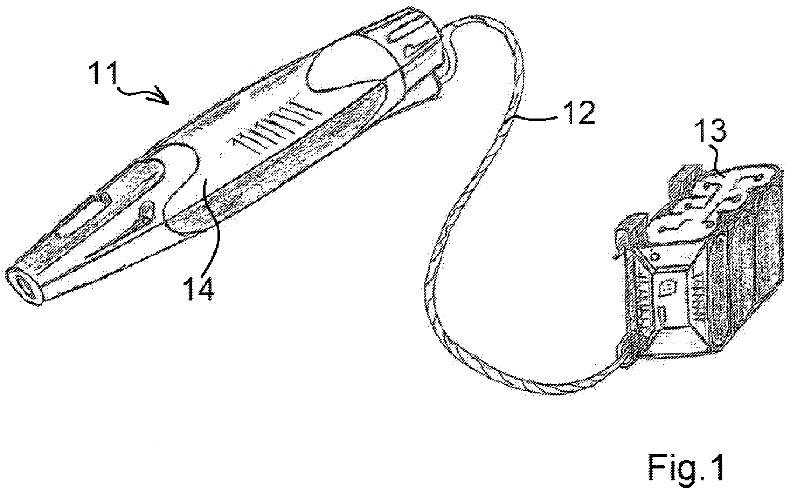

In Fig. 1 ist mit 10 in ihrer Gesamtheit eine Injektionsvorrichtung nach der Erfindung bezeichnet, die ein Handteil 11 aufweist, das über eine Kabelverbindung 12 mit einer externen Stromversorgung 13 , im gezeigten Ausführungsbeispiel einem Akkupack, verbunden ist.

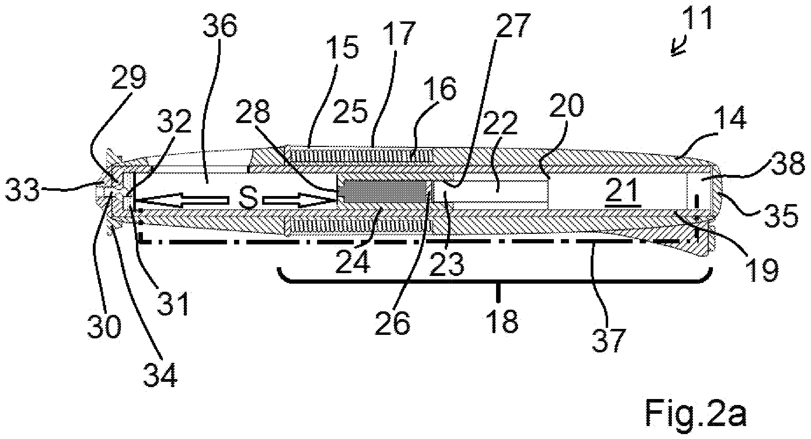

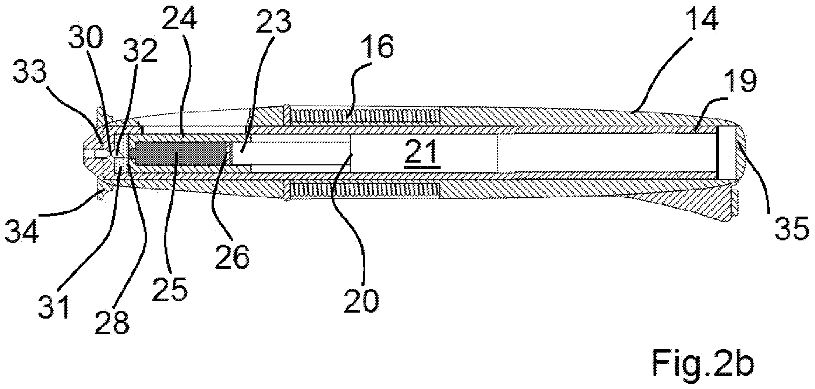

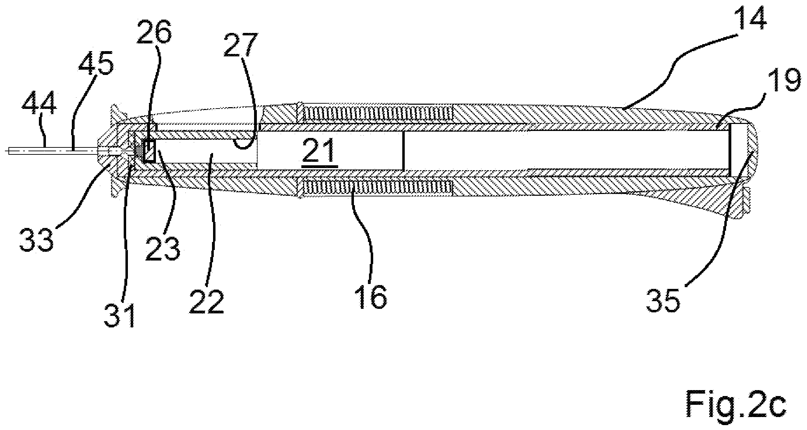

Das Handteil 11 der Injektionsvorrichtung 10 kann von seinem Benutzer bequem mit einer einzigen Hand gehandhabt werden. Der nähere Aufbau des Handteils 11 ist in der Schnittdarstellung gemäß den Fig. 2 a bis c gut zu erkennen. Demnach weist es ein Gehäuse 14 auf, das an seinem Außenumfang mit einer Aussparung 15 versehen ist, in der eine Magnetspule 16 aufgenommen ist. Die Magnetspule 16 ist mittels einer umlaufenden Abdeckung 17 geschützt.

Die Magnetspule 16 ist Bestandteil einer insgesamt mit 18 bezeichneten Ejektoreinrichtung, zu der weiter ein in das Gehäuse eingestecktes, dieses im Wesentlichen von seinem hinteren Ende (in der Zeichnung rechts) bis zu dem vorderen (links) Auslassende durchsetzendes Ejektorrohr 19 aus Kunststoff und ein in diesem längsverschieblich geführter Ejektorstößel 20 gehören, der bei dem gezeigten Ausführungsbeispiel einen hinteren Abschnitt 21 und einen vorderen Abschnitt 22 aufweist. Während der hintere Abschnitt mit größerem Durchmesser an den Innenquerschnitt des Ejektorrohrs 19 angepasst ist und weitestgehend spiel- und reibungsfrei in diesem gleiten kann, weist der vordere Abschnitt 22 einen geringeren Durchmesser auf. Er bildet ein Druckstück 23, das von hinten in ein zylindrisches Flüssigkeitsbehältnis 24 in Form einer Flüssigkeitspatrone oder -kartusche einschiebbar bzw. eingeschoben ist, das eine in ein Substrat, beispielsweise in oder unter die Haut eines Menschen oder Tiers, zu injizierende Flüssigkeit 25 enthält. Dieses Flüssigkeitsbehältnis 24 ist ähnlich wie der hintere Abschnitt 21 des Ejektorstößels 20 in dem Ejektorrohr 19 im Wesentlichen spielfrei aufgenommen, so dass es ebenfalls leicht in diesem gleiten kann. Rückseitig (in der Zeichnung also rechts) ist das Flüssigkeitsbehältnis 24 mit einem Kolben 26 verschlossen, der die Flüssigkeit 25 im Behältnis 24 hält und so weit in den von dem Behältnis 24 definierten Zylinderraum 27 eingeschoben ist, dass an seiner Rückseite das Druckstück 23 ebenfalls ein Stück weit in diesen Zylinderraum einfasst. An seiner in der Zeichnung linken Auslassseite ist das Flüssigkeitsbehältnis 24 mit einer Membran 28 verschlossen.

Das Ejektorrohr 19 ist an seiner vorderen, in Fig.2 linken Stirnseite mit einer Kappe 29 versehen, die eine zentrale Öffnung aufweist, in der eine nach innen in Richtung auf das Flüssigkeitsbehältnis 24 vorspringende Aufstechkanüle 30 aufgenommen ist. Innen an der Kappe ist ein die Aufstechkanüle 30 umgebendes, elastisches Pufferelement 31 angeordnet.

Die Aufstechkanüle 30 steht mit ihrem ihrer Aufstechspitze 32 gegenüberliegenden, auslassseitigen Ende ein Stück weit über die Kappe 30 vor und bildet hierdurch eine Zentrierung für eine Auslassdüse 33, die auf dieses auslassseitige Ende der Kanüle 31 aufgesteckt und am Gehäuse 14 mittels einer Überwurfmutter 34 festgelegt ist.

Um die Vorrichtung zur Benutzung vorzubereiten, wird zunächst der Ejektorstößel 20 mit seinem vorderen, das Druckstück 23 bildenden Abschnitt 22 von hinten in das patronenartig ausgestaltete Flüssigkeitsbehältnis 24 eingesteckt, wobei sich die Stirnseite des Druckstücks 23 an den Kolben 26 in der zylindrischen Öffnung des Flüssigkeitsbehältnisses anlegt. Diese Baueinheit aus Flüssigkeitspatrone und Ejektorstößel kann dann mit der die Flüssigkeitspatrone vorne verschließenden Membran 28 voran von hinten in das Ejektorrohr 19 im Gehäuse 16 eingeschoben werden, wozu eine rückwärtig am Gehäuse angeordnete Abdeckkappe 35 geöffnet werden kann. Nach Schließen der Abdeckkappe ist die Vorrichtung betriebsbereit. Dieser Betriebszustand ist in Fig. 2a gezeigt.

Anhand der Fig.2a bis 2c lässt sich die Wirkungsweise der erfindungsgemäßen Vorrichtung bei einem Injektionsvorgang gut nachvollziehen: Fig. 2a zeigt dabei die Ausgangsstellung der Ejektoreinrichtung, bei der sich der hintere Abschnitt 21 des Ejektorstößels 20 in der am weitesten hinten gelegenen Position (in der Zeichnung rechts) befindet (hintere Endstellung). Der in dieser Stellung des Ejektorstößels und des vorne auf diesen aufgesteckten Flüssigkeitsbehältnisses vor diesem bis zur endseitigen Kappe 29 sich erstreckende Freiraum in dem Ejektorrohr 19 bildet eine Beschleunigungsstrecke S, auf deren Länge die aus Stößel und Flüssigkeitsbehältnis 24 bestehende Baueinheit beschleunigt werden kann. Um aus der in Fig.2a gezeigten Stellung einen Injektionsvorgang auszulösen, wird die Magnetspule 16 mit elektrischer Energie aus dem Akkupack 13 bestromt und beschleunigt hierdurch den Ejektorstößel 20 mit der daran vorne aufgesteckten Flüssigkeitspatrone 24 über die Beschleunigungsstrecke S in einer Bewegungsrichtung in Richtung auf die Auslassdüse (in der Zeichnung nach links). Dabei erreicht die beschleunigte Baueinheit in kürzester Zeit eine sehr hohe Geschwindigkeit, die in der Praxis über 500 m/s, bei geeignet längerer Beschleunigungsstrecke sogar über 800 m/s betragen kann. Diese Bewegung macht das Flüssigkeitsbehältnis 24 mit der darin aufgenommenen Flüssigkeit 25 zunächst mit, bis es von dem Pufferelement 31 abgebremst wird, das zwischen der vorderen Kappe 29 des Ejektorrohrs 19 und der mit großer Geschwindigkeit von der Magnetspule 16 in Richtung auf die Auslassdüse 33 bewegten Baueinheit aus Ejektorstößel 20 und Flüssigkeitsbehältnis 24 zusammengedrückt wird. Das Pufferelement 31 dient vor allem dazu, zu verhindern, dass das gegen die vordere Kappe 29 anschlagende Flüssigkeitsbehältnis von diesem wieder zurückspringt. Die Stellung der Ejektoreinrichtung in diesem Betriebszustand ist in Fig. 2b gezeigt.

Wie in der Darstellung gemäß Fig.2a lediglich schematisch in strichpunktierten Linien angedeutet ist, ist der Freiraum 36, der im Inneren des Ejektorrohrs 19 zwischen dessen vorderer Kappe 29 und dem stirnseitigen, von der Membran verschlossenen Ende des Flüssigkeitsbehältnisses 24 vorhanden ist, mittels einer Überströmleitung 37 mit dem Raum 38 hinter dem hinteren Stößelende 21 verbunden. Durch die Überströmleitung kann Luft aus dem vorderen Freiraum 36 verdrängt bzw. tatsächlich aktiv durch den sich hinter dem Stößel bei dessen Vorwärtsbewegung bildenden Unterdruck im Raum 38 abgesaugt werden, womit sichergestellt ist, dass der Ejektorstößel 20 mit dem Flüssigkeitsbehältnis 24 nicht infolge erhöhten Luftwiderstands abgebremst wird. In der praktischen Umsetzung dieses Merkmals kann die Überströmleitung in der Wandung des Gehäuses integriert sein, so dass sie von außen tatsächlich nicht weiter auffällt.

Sobald die Aufstechspitze 32 der Aufstechkanüle 30 die am vorderen Ende des Flüssigkeitsbehältnisses 24 vorgesehene Membran 28 durchsticht, kann die in dem Behältnis aufgenommene Flüssigkeit 25 aus diesem vorne austreten und durch die Kanüle 31 in die Auslassdüse 33 gelangen. Da sich im Moment des Aufstechens das Flüssigkeitsbehältnis 24 mit der darin aufgenommenen Flüssigkeit 25 immer noch mit hoher Geschwindigkeit bewegt und diese Bewegung sehr abrupt zum Abbruch kommt, sobald das Pufferelement 29 maximal möglich zusammengepresst ist, kommt es zu einer kurzfristigen, starken Druckerhöhung in dem im Behältnis 24 aufgenommenen Flüssigkeitsvolumen (Druckstoß), denn der hinten auf den Kolben 26 im Flüssigkeitsbehältnis 24 mit seinem Druckstück 23 drückende Ejektorstößel 20 wird genauso plötzlich abgebremst und überträgt die ihm innewohnende dynamische Energie als Impulsstoß in die zunächst mitbeschleunigte Flüssigkeit, was den starken Druckanstieg in dieser auslöst. Aufgrund dieses kurzfristig hohen Druckes in der Flüssigkeit wird eine erste Teilmenge der Flüssigkeit mit entsprechend hohem Druck durch die Kanüle und die sich anschließende Auslassdüse 33 gepresst und verlässt diese wiederum mit einer dem hohen statischen Druck entsprechenden großen Mündungsgeschwindigkeit an der Auslassseite der Auslassdüse, wo der Flüssigkeit Umgebungsdruck aufgeprägt wird und die ihr innewohnende Druckenergie in kinetische Energie (Geschwindigkeit) umgesetzt wird. In der Praxis kann die zum Einsatz gebrachte Auslassdüse, die bevorzugt wie nachstehend noch beschrieben gestaltet ist, einen Durchlass 36 für die Flüssigkeit 25 mit einem Durchmesser von 80 bis 300 µm aufweisen, so dass die erste, infolge des Impulsstoßes ausgestoßene Teilmenge Flüssigkeit als sehr feiner Flüssigkeitsstrahl mit entsprechend geringem Querschnitt auf das Substrat mit sehr großer Geschwindigkeit auftrifft. Die Austrittsgeschwindigkeit der Flüssigkeit infolge des Druckstoßes kann dabei ohne weiteres 1000 m/s betragen. Mit diesem extrem schnellen und dünnen Flüssigkeitsstrahl wird in dem Substrat ein Injektionskanal bis in eine Tiefe erzeugt (geschossen), die abhängig ist von der Strahlgeschwindigkeit und seinem Durchmesser und damit letztendlich von der Stärke des Impulsstoßes, der von dem Ejektorstößel in dem Flüssigkeitsvorrat erzeugt wird.

Erfindungsgemäß ist es möglich, die gesamte im Flüssigkeitsbehältnis enthaltene Flüssigkeitsmenge oder jedenfalls zusätzlich zu der ersten, wie vorstehend erläutert unter Bildung eines Injektionskanals injizierten Teilmenge eine zweite Teilmenge Flüssigkeit an diesem Injektionsort in das Substrat zu injizieren., Hierzu kann die Magnetspule 16 nach Erreichen der vorderen Endstellung des Flüssigkeitsbehältnisses 24 (Fig. 2b) weiter bestromt werden. Dadurch wird der Ejektorstößel 20 mit dessen vorderen Abschnitt 22 (Druckstück 23) weiter von hinten gegen den Kolben 26 im Flüssigkeitsbehältnis gedrückt, so dass zumindest ein Teil der nach dem Abbau des Druckstoßes im Behältnis noch verbliebenen Flüssigkeit (zweite Teilmenge) wie bei einer herkömmlichen Spritze durch die Kanüle 31 gedrückt und anschließend durch die Auslassdüse 33 ausgestoßen wird. Es hat sich überraschend gezeigt, dass trotz des dann deutlichen geringeren Drucks, mit der die zweite Teilmenge ejiziert wird, und der daraus resultierenden geringeren Auslassgeschwinddigkeit der zweiten Teilmenge Flüssigkeit aus der Auslassdüse auch die zweite Teilmenge zuverlässig und vollständig in den mittels der ersten Teilmenge vorab in dem Substrat erzeugten Injektionskanal eindringt und somit in das Substrat, also bei dem Ausführungsbeispiel in oder unter die Haut gelangt. Dabei kommt es im Allgemeinen am Ende des Injektionskanals zu einer Depotbildung, d.h. die zweite Teilmenge Flüssigkeit verteilt sich im Wesentlichen gleichmäßig in dem Gewebe kugelförmig um den Endbereich des Injektionskanals herum. Der Injektionsvorgang kann so lange fortgesetzt werden, bis der Kolben 26 von dem Druckstück 23 vollständig bis zum vorderen Ende des Flüssigkeitsbehältnisses eingeschoben ist (Fig. 2c).

Wenn es gewünscht wird, kann mit der Vorrichtung auch eine Abfolge von mehr oder weniger dicht beieinander positionierten Injektionen von jeweils vergleichsweise kleinen Flüssigkeitsmengen gesetzt werden, und zwar in kurzen zeitlichen Abständen. Hierzu wird durch geeignete Ansteuerung (Änderung der Stromflussrichtung) der Magnetspule 16 der Ejektorstößel 20 direkt nach Erzeugen eines Impulsstoßes in der im Behältnis aufgenommenen Flüssigkeit wieder zurück in seiner Ausgangsstellung gezogen (also in der Zeichnung nach rechts). Da das Flüssigkeitsbehältnis 24 bei dem hier beschriebenen Ausführungsbeispiel nach dem allerersten, wie vorstehend beschrieben durchgeführten Injektionsvorgang von der Aufstechspitze 32 der Kanüle 30 an der Membran bereits geöffnet ist, verbleibt es bei dieser Betriebsweise zweckmäßig in seiner in der Zeichnung gemäß Fig.2c gezeigten linksseitigen Endstellung, was durch ein geeignetes, nicht dargestelltes Rückhalteelement gewährleistet werden kann. Beispielsweise kann zu diesem Zweck ein quer zur Längsachse des Ejektorrohrs 19 mit einer Feder radial nach innen vorgespannter Riegel in einer Aussparung im Ejektorrohr aufgenommen sein, der nach dem Vorbeigang der Flüssigkeitspatrone nach Auslösen des ersten Injektionsvorgangs sich unter dem Federdruck radial nach innen bewegt und dabei hinter den rückwärtigen (in der Zeichnungen am rechten Ende des Flüssigkeitsbehältnisses) Rand des Flüssigkeitsbehältnisses fasst und so verhindert, dass sich dieses wieder zurück bewegen kann. Der durch kurzfristige Umpolung der Magnetspule wieder zurückgezogenen Ejektorstößel 20 kann in seiner wieder zurückgezogenen Stellung mittels eines kleinen Permanentmagneten oder eines Elektromagneten an der rückwärtigen Abdeckkappe 35 des Gehäuses gehalten werden, so dass er nicht unbeabsichtigt und/oder verfrüht allein infolge seines Eigengewichts wieder gegen das Stoßeinleitungselement (Kolben 26) am Flüssigkeitsbehältnis fällt. Der Ejektorstößel kann dann, ggf. unter Überwindung der Magnethaltekraft des erwähnten (nicht gezeigten) Permanent- oder Elektromagneten, erneut über die vor ihm liegende Beschleunigungsstrecke auf hohe Geschwindigkeit beschleunigt werden, wobei er zum Ende seiner Bewegung mit dem vorderen Druckstück wieder in den Zylinderraum am rückwärtigen Ende des Flüssigkeitsbehältnisses hineingleitet und dort auf dem Kolben 26 aufschlägt und somit erneut einen Druckstoß zur Ejektion einer weiteren (kleinen) Teilmenge Flüssigkeit erzeugt. Die wiederholte Auslösung des Elektromagneten und damit bewirkte Ejektion von Flüssigkeit aus der Vorrichtung kann (nach deren Neupositionierung an der nächsten, gewünschten Injektionsstelle) dabei manuell, also durch Betätigung eines (nicht gezeigten) Auslösemechanismus erfolgen, oder auch automatisch in vorher festgelegten Zeitabständen, wobei diese, beispielsweise bei Verwendung der Vorrichtung als Tätowiergerät, auch sehr kurz gewählt werden können. Ein Betrieb der Vorrichtung mit einer Auslösefrequenz in der Größenordnung von 35 bis 200 Hz ist bei geeigneter Dimensionierung des Stößels und der Beschleunigungsstrecke ohne weiteres möglich.

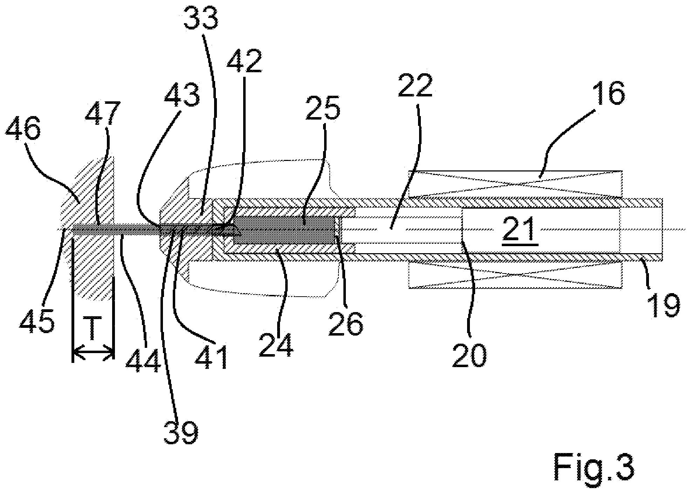

In Fig. 3 ist in ihrem Einbauzustand am Gehäuse der erfindungsgemäßen Vorrichtung eine erste bevorzugte Ausführungsform der zum Einsatz kommenden Auslassdüse 33 dargestellt. Man erkennt, dass diese Auslassdüse 33 einen zentralen, koaxial zu der Kanüle 30 verlaufenden Durchlass 39 für die zu injizierende Flüssigkeit 25 aufweist, der an seiner Durchlasswandung 40 mindestens einen schraubengang- bzw. wendelförmigen Fluidkanal 41 aufweist, der sich vom Düseneinlass 42 an der Seite der Kanüle 30 bis zum Düsenauslass 43 erstreckt, aus dem die Flüssigkeit 25 zur Injektion austritt. Dieser schraubengangförmige Fluidkanal 41 bewirkt, dass der durch die Auslassdüse 33 strömenden Flüssigkeit ein Drall bzw. eine Drehbewegung aufgeprägt wird, so dass der Flüssigkeitsstrahl 44 bei Austritt aus der Düse in Rotation um seine Strahlachse 45 versetzt ist/wird und somit als rotierender Flüssigkeitsstrahl auf das Substrat 46, im Ausführungsbeispiel die Haut eines Menschen oder Tiers auftrifft.

Die Überlagerung der translatorischen Bewegung der Flüssigkeit mit der ihm aufgeprägten Rotation bewirkt, dass sich der Flüssigkeitsstrahl 44 beim Auftreffen auf das Substrat 46 praktisch in dieses hineinschraubt oder hineinbohrt, wobei die schraubenartige Bewegung der Flüssigkeit den Strahl offenbar in sich zusammenhält, so dass es beim Auftreffen auf die Haut- bzw. Substratoberfläche nicht zu einem Aufpilzen und seitlichen Wegspritzen von Flüssigkeit kommt, sondern diese jedenfalls weitestgehend verlustfrei in das Substrat eintritt und in diesem einen Injektionskanal 47 mit einer Tiefe T erzeugt, die im Wesentlichen von der Beschaffenheit des Substrats, der Geschwindigkeit des Flüssigkeitsstrahls in Axialrichtung sowie seinem Querschnitt abhängt. Bei dem gezeigten Ausführungsbeispiel hat der Durchlass 39 in der Auslassdüse auslassseitig einen Durchmesser von ca. 80 bis 100 µm und der infolge des Druckstoßes im Flüssigkeitsvorrat diesen verlassende (erste) Teilstrom tritt mit einer Geschwindigkeit in der Größenordnung von 100 bis 1000 m/s aus der Düse aus. Die Tiefe des daraus resultierenden Injektionskanals in (menschlichem oder tierischem) Gewebe kann damit zwischen wenigen Millimetern und einigen Zentimetern eingestellt werden.

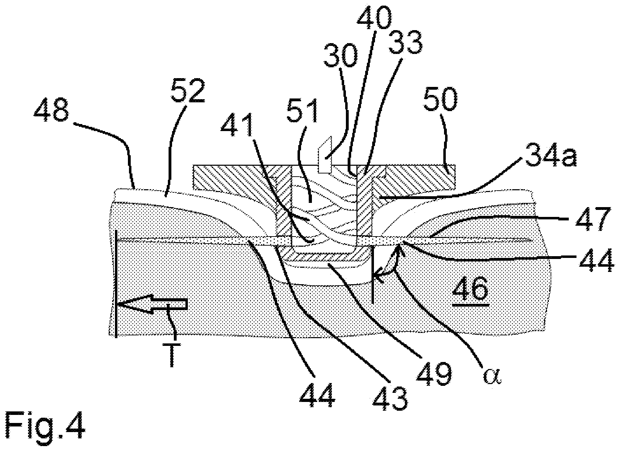

Fig. 4 zeigt eine weitere Ausführungsform einer erfindungsgemäßen Auslassdüse, wobei entsprechende Merkmale mit denselben Bezugszeichen wie bei der ersten Ausführungsform versehen sind. Die in Fig. 4 gezeigte Auslassdüse 33 ist mittels einer Überwurfmutter 34a am Gehäuse festgelegt, die zugleich einen Abstandhalter bzw. eine Tiefenlehre bildet. Die Auslassdüse gemäß Fig.4 kann ein Stück weit in das Substrat 46, nämlich von deren Oberseite 48 in die Haut eines Patienten eingedrückt werden, so dass sie darin einen muldenartige Vertiefung 49 ausbildet. Ein radial nach außen vorspringender Ringbereich 50 an der Überwurfmutter 34a begrenzt dabei die Eindrücktiefe der Düse bzw. zeigt das Erreichen einer gewünschte Tiefe an, was dann der Fall ist, wenn der äußere Rand des Ringbereichs 50 ebenfalls in Kontakt mit der Hautoberfläche 48 gelangt. Die Auslassdüse 33 hat einen Durchlass 39 mit einer einlassseitig etwa tassenförmigen Düsenkammer 51, an deren Wandung zwei (oder mehr) Fluidkanäle 41 ausgebildet sind, die sich nach Art einer Doppel- (oder Mehrfach-)Helix schraubengangförmig umschlingen und die wie beschrieben der durch die Düse strömenden Flüssigkeit den erfindungsgemäßen Drall (Schraubenbewegung) aufprägen. Die Düse hat zwei (oder auch mehrere) seitlich etwa radial nach außen offene Düsenauslässe 43, durch die Flüssigkeitsstrahlen 44 anders als bei der ersten Ausführungsform der Vorrichtung die Düse nicht koaxial zu deren Längsrichtung, sondern in Richtungen verlassen, die im Wesentlichen senkrecht zur Längsachse der Vorrichtung oder - im gezeigten Ausführungsbeispiel - sogar einem Winkel a verlaufen, der geringfügig größer als 90° sein kann. Auf diese Weise ist es leicht möglich, die Flüssigkeit nicht senkrecht zur Substratoberfläche zu injizieren, sondern sie unter die oberste Hautschicht 52 im Wesentlichen parallel zu dieser im Substrat zu verteilen.

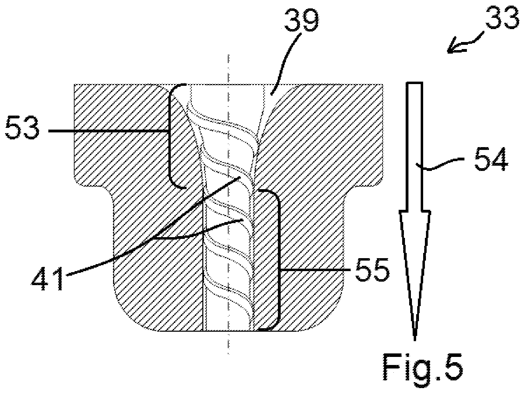

Die in Fig. 5 dargestellte Ausführungsform einer Auslassdüse 33 stimmt weitgehend mit der nach Fig.3 überein. Allerdings hat der Durchlass 39 hier keinen konstanten Querschnitt über seine gesamte Länge, sondern er weist einlassseitig zunächst einen konvergierenden Abschnitt 53 auf, dessen Querschnitt sich in Durchströmrichtung 54 der durch die Düse ejizierten Flüssigkeit 25 verringert, um dann in einen Abschnitt konstanten Querschnitts 55 überzugehen. In beiden Abschnitten 53 und 55 sind an deren Wandungen sich schraubengangförmig wendelnde Fluidkanäle 41 vorgesehen, bei dem gezeigten Ausführungsbeispiel zwei Kanäle, die nach Art einer Doppelhelix angeordnet sind. Der konvergierende Abschnitt sorgt zunächst für eine Beschleunigung der aus dem Flüssigkeitsbehältnis in die Düse gelangenden Flüssigkeit.

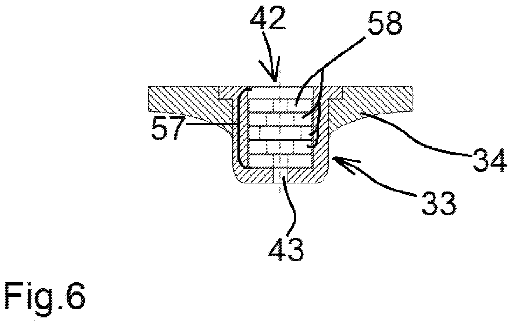

Fig. 6 zeigt eine Ausführungsform der Auslassdüse für die Erfindung, bei der der oder ein Fluidkanal 41 sich in Form einer gewendelten Rohrleitung 56 von der Einlass- 42 zur Auslassseite 43 der Auslassdüse 33 durch diese erstreckt. Dabei ist die Anordnung so getroffen, dass die Rohrleitung 56 einen sich von der Einlassseite 42 zur Auslassseite 43 hin abnehmenden Wendelradius R aufweist. Hierdurch wird ein Zykloneffekt erreicht, also eine Beschleunigung der Rotationsgeschwindigkeit der durch die Rohrwendel 56 strömenden Flüssigkeit um sich selbst, so dass die Flüssigkeit bei ihrem Austritt am Düsenauslass mit hoher Geschwindigkeit um sich selbst rotiert.

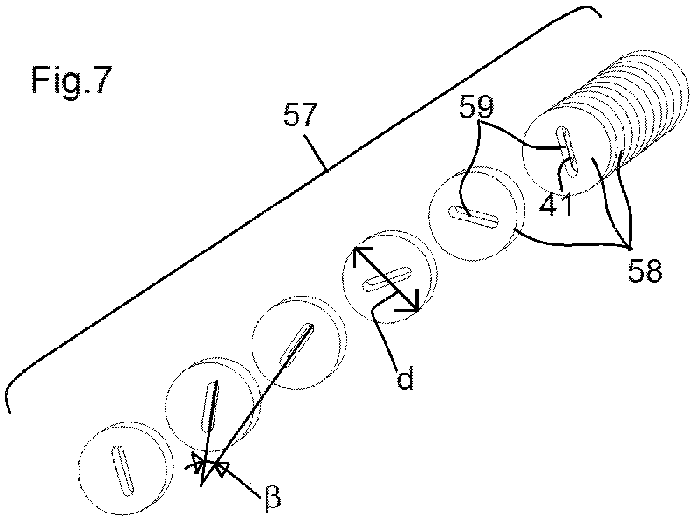

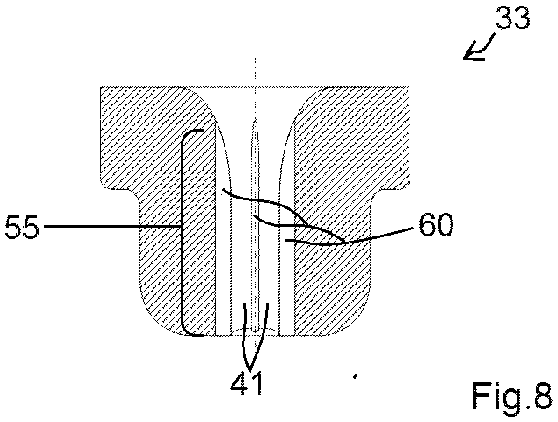

Bei der in den Fig. 7 und 8 gezeigten Ausführungsform hat die Auslassdüse 33 mehrere in Durchlassrichtung 54 der Flüssigkeit hintereinander in Form eines Blendenstapels 57 angeordnete Blendenscheiben 58, die jeweils eine sich über einen Teil des Scheibendurchmessers d erstreckende Schlitzöffnung 59 aufweisen, wobei die Schlitzöffnungen 59 von in dem Blendenstapel 57 aufeinander folgenden Blendenscheiben 58 in Umfangsrichtung um einen Winkelbetrag β zueinander versetzt angeordnet sind. Der Betrag dieses Winkelversatzes β in Umfangsrichtung ist an den radial äußeren Enden der Schlitzöffnungen 59 kleiner ist als die Breite der Schlitzöffnungen. Hierdurch ergibt ein wendeltreppenartiger Fluidkanal 41 mit einer zentralen Durchlassöffnung. Die Ausführungsform mit den gestapelten Blenden ist auch mit kleinsten Abmessungen mit einem Durchlassquerschnitt, der im Mikrometerbereich liegt, besonders einfach und kostengünstig herstellbar.

Bei der in Fig. 8 gezeigten Auslassdüse 33 sind an der Wandung 40 des sie durchsetzenden Durchlasses 39 vier Fluidkanäle 41 ausgebildet, die über die Länge des Abschnitts mit konstantem Querschnitt 55 geradlinig parallel zur Durchströmrichtung verlaufen und die durch Stege 60 gegeneinander abgetrennt sind. Die gesamte Düse ist bei dieser Ausführungsform drehbar am Gehäuse der Vorrichtung gelagert und mittels einer Spule elektromotorisch antreibbar. Wenn Sie während des Ejektionsvorgangs in Drehung versetzt wird, übertragen die Stege an der der Durchlasswandung diese Drehbewegung in den äußeren Umfangsbereich des durch die Düse strömenden Flüssigkeitsstrahls und prägen diesem somit die erfindungsgemäße Drehbewegung auf.

## Patentansprüche

1. Injektionsvorrichtung für die nadellose Injektion einer Flüssigkeit (25) in ein Substrat (46), insbesondere zur Injektion von flüssigem pharmazeutischen oder kosmetischen Präparat in ein biologisches Gewebe, mit einem Gehäuse (14), einem im Gehäuse aufgenommenen oder anordbaren Flüssigkeitsvorrat (24), einer Auslassdüse (33) und mit einer Ejektionseinrichtung (18) zum Ejizieren von Flüssigkeit (25) aus dem Flüssigkeitsvorrat (24) durch die Auslassdüse (33), wobei die Ejektionseinrichtung (18) einen auf eine Stoßgeschwindigkeit beschleunigbaren Ejektionsstößel (20) zum Erzeugen eines auf wenigstens eine erste Teilmenge Flüssigkeit (25) im Flüssigkeitsvorrat (24) wirkenden Impulsstoßes aufweist, **dadurch gekennzeichnet, dass** eine Beschleunigungsstrecke des Ejektionsstößels (20) im Bereich vor und hinter dem Ejektionsstößel (20) mit Druckausgleichsöffnungen in Verbindung ist, sodass ein Einfluss von Luftkompression bzw. eines entstehenden Unterdrucks in der Beschleunigungsstrecke auf die Bewegung des Ejektionsstößels (20) weitestgehend unterbunden werden kann.

2. Vorrichtung nach Anspruch 1, **dadurch gekennzeichnet, dass** der Flüssigkeitsvorrat (24) mittels eines von der Ejektionseinrichtung betätigbaren Ejektionskolbens (26) beaufschlagbar ist, welcher wiederum von dem Ejektionsstößel (20) beaufschlagbar oder von diesem gebildet wird.

3. Vorrichtung nach Anspruch 1 oder 2, **dadurch gekennzeichnet, dass** die Ejektionseinrichtung (18) einen elektromagnetischen Antrieb (16) für den Ejektionsstößel (20) aufweist.

4. Vorrichtung nach einem der Ansprüche 1 bis 3, **dadurch gekennzeichnet, dass** die Ejektionseinrichtung (18) die Beschleunigungsstrecke (S) für den Ejektionsstößel (20) aufweist.

5. Vorrichtung nach Anspruch 3 oder 4, **dadurch gekennzeichnet, dass** der elektromagnetische Antrieb (16) an einem von der Auslassdüse (33) beabstandeten, rückwärtigen Ende des Gehäuses oder etwa in dessen Mitte angeordnet ist und sich die Beschleunigungsstrecke (S) zwischen der Auslassdüse und dem rückwärtigen Gehäuseende erstreckt.

6. Vorrichtung nach Anspruch 3 oder 4, **dadurch gekennzeichnet, dass** der elektromagnetische Antrieb (16) eine am Ejektionsstößel (20) selbst ausgebildete Magnetspule sowie einen den Ejektionsstößel (20) umgebenden Eisenzylinder und/oder eine Ständerspule aufweist.

7. Vorrichtung nach einem der Ansprüche 3 bis 6, **dadurch gekennzeichnet, dass** der Ejektionsstößel (20) mit einer Stromspeichereinrichtung versehen ist, um den elektromagnetischen Antrieb (16) mit Strom zu versorgen.

8. Vorrichtung nach Anspruch 1, **dadurch gekennzeichnet, dass** die Druckausgleichsöffnungen über eine Überströmleitung (37) miteinander verbunden sind.

9. Vorrichtung nach einem der vorangegangenen Ansprüche, **dadurch gekennzeichnet, dass** die Ejektionseinrichtung (18) Mittel zum Erzeugen einer Druckerhöhung im Flüssigkeitsvorrat (24) in Anschluss an den ausgeübten Impulsstoß aufweist.

10. Vorrichtung nach Anspruch 9, **dadurch gekennzeichnet, dass** die Mittel zum Erzeugen einer Druckerhöhung im Wesentlichen von dem Ejektionsstößel (20) gebildet werden, der nach Ausübung des Impulsstoßes mittels eines kraftausübenden Antriebs (16) auf den Flüssigkeitsvorrat wirkt.

11. Vorrichtung nach Anspruch 10, **dadurch gekennzeichnet, dass** der kraftausübende Antrieb (16) ein elektromagnetischer Antrieb (16) für den Ejektionsstößel (20) ist.

12. Vorrichtung nach einem der vorangegangenen Ansprüche, **dadurch gekennzeichnet, dass** der Flüssigkeitsvorrat in einem Flüssigkeitsbehältnis (24) aufgenommen ist, das bevorzugt austauschbar im Gehäuse (14) anordbar ist.

13. Vorrichtung nach einem der vorangegangen Ansprüche, **dadurch gekennzeichnet, dass** das Flüssigkeitsbehältnis (24) mit der darin aufgenommenen Flüssigkeit (25) zusammen mit dem Ejektionsstößel (20) in dem Gehäuse (14) bzw. der darin vorgesehenen Beschleunigungsstrecke (S) beweglich aufgenommen oder aufnehmbar ist und dass das Gehäuse (14) an seinem vorderen Auslassende einen Anschlag (30) für das Flüssigkeitsbehältnis (24) aufweist.

14. Vorrichtung nach Anspruch 26, **dadurch gekennzeichnet, dass** der Anschlag (30) und/oder das Flüssigkeitsbehältnis (24) mit einem Anschlagdämpfer (29), beispielsweise einem Elastomer-Pufferelement versehen ist/sind.

15. Vorrichtung nach einem der vorangegangenen Ansprüche, in der ein Flüssigkeitsbehältnis angeordnet ist, das mindestens einen eine erste in ein Substrat zu injizierende Flüssigkeit aufnehmenden Kolbenraum aufweist, der einen Flüssigkeitsauslass und ein Stoßeinleitungselement zur Einleitung eines auf das Flüssigkeitsbehältnis ausübbaren Impulsstoßes aufweist.

## Claims

1. An injection device for the needleless injection of a liquid (25) into a substrate (46), in particular for the injection of liquid pharmaceutical or cosmetic preparation into a biological tissue, comprising a housing (14), a liquid supply (24) accommodated or arrangeable in the housing, an outlet nozzle (33) and an ejection device (18) for ejecting liquid (25) from the liquid supply (24) through the outlet nozzle (33), the ejection device (18) having an ejection plunger (20) which can be accelerated to an impact velocity for generating a pulse impact acting on at least a first partial quantity of liquid (25) in the liquid supply (24), **characterized in that** an acceleration path of the ejection plunger (20) in the region upstream and downstream of the ejection plunger (20) is connected to pressure compensation openings, so that an influence of air compression or of a resulting underpressure in the acceleration path of the ejection plunger (20) is prevented.

2. The device according to claim 1, **characterized in that** the liquid supply (24) can be acted upon by means of an ejection piston (26) which can be actuated by the ejection device and which in turn can be acted upon by the ejection plunger (20) or is formed by the latter.

3. The device according to claim 1 or 2, **characterized in that** the ejection device (18) comprises an electromagnetic drive (16) for the ejection plunger (20).

4. The device according to any one of claims 1 to 3, **characterized in that** the ejection device (18) comprises the acceleration path (S) for the ejection plunger (20).

5. The device according to claim 3 or 4, **characterized in that** the electromagnetic drive (16) is arranged at a rear end of the housing spaced from the outlet nozzle (33) or approximately in the center thereof and the acceleration path (S) extends between the outlet nozzle and the rear end of the housing.

6. The device according to claim 3 or 4, **characterized in that** the electromagnetic drive (16) comprises a solenoid coil formed on the ejection plunger (20) itself and an iron cylinder surrounding the ejection plunger (20) and/or a stator coil.

7. The device according to any one of claims 3 to 6, **characterized in that** the ejection plunger (20) is provided with a current storage device for supplying current to the electromagnetic drive (16).

8. The device according to claim 1, **characterized in that** the pressure equalization openings are connected to one another via an overflow line (37).

9. The device according to any one of the preceding claims, **characterized in that** the ejection device (18) comprises means for generating an increase in pressure in the fluid reservoir (24) following the applied impulse shock.

10. The device according to claim 9, **characterized in that** the means for generating an increase in pressure are substantially constituted by the ejection plunger (20) which, after the impulse is exerted, acts on the liquid supply by means of a force-exerting drive (16).

11. The device according to claim 10, **characterized in that** the force exerting drive (16) is an electromagnetic drive (16) for the ejection plunger (20).

12. The device according to any one of the preceding claims, **characterized in that** the liquid supply is accommodated in a liquid container (24) which is preferably exchangeably arrangeable in the housing (14).

13. The device according to one of the preceding claims, **characterized in that** the liquid container (24) with the liquid (25) received therein is movably received or receivable together with the ejection plunger (20) in the housing (14) or the acceleration section (S) provided therein, and **in that** the housing (14) has a stop (30) for the liquid container (24) at its front outlet end.

14. The device according to claim 26, **characterized in that** the stop (30) and/or the liquid container (24) is/are provided with a stop damper (29), for example an elastomer buffer element.

15. The device according to any one of the preceding claims, wherein there is disposed a fluid reservoir having at least one piston chamber receiving a first fluid to be injected into a substrate, the piston chamber having a fluid outlet and a shock introducing member for introducing a pulse shock exertable on the fluid reservoir.

## Revendications

1. Dispositif d'injection pour une injection sans aiguille d'un liquide (25) dans un substrat (46), plus particulièrement pour l'injection d'une préparation pharmaceutique ou cosmétique fluide dans un tissu biologique, avec un boîtier (14), un réservoir de liquide (24) logé ou pouvant être disposé dans le boîtier, une buse de sortie (33) et un dispositif d'éjection (18) pour l'éjection d'un liquide (25) hors du réservoir de liquide (24) à travers la buse de sortie (33), dans lequel le dispositif d'éjection (18) comprend un poussoir d'éjection (20) pouvant être accéléré à une vitesse d'impact, afin de générer une impulsion agissant sur au moins une première quantité partielle de liquide (25) dans le réservoir de liquide (24), **caractérisé en ce qu'**une trajectoire d'accélération du poussoir d'éjection (20) est en liaison, dans la partie à l'avant et à l'arrière du poussoir d'éjection (20), avec des ouvertures de compensation de pression, de sorte qu'une influence de la compression de l'air ou d'une dépression qui en résulte dans la trajectoire d'accélération sur le mouvement du poussoir d'éjection (20) peut être largement évitée.

2. Dispositif selon la revendication 1, **caractérisé en ce que** le réservoir de liquide (24) peut être sollicité au moyen d'un piston d'éjection (26), pouvant être actionné par le dispositif d'éjection, qui peut à son tour être sollicité par le poussoir d'éjection (20) ou qui est constitué de celui-ci.

3. Dispositif selon la revendication 1 ou 2, **caractérisé en ce que** le dispositif d'éjection (18) comprend un entraînement magnétique (16) pour le poussoir d'éjection (20).

4. Dispositif selon l'une des revendications 1 à 3, **caractérisé en ce que** le dispositif d'éjection (18) comprend la trajectoire d'accélération (S) pour le poussoir d'éjection (20).

5. Dispositif selon la revendication 3 ou 4, **caractérisé en ce que** l'entraînement magnétique (16) est disposé au niveau d'une extrémité arrière, distante de la buse de sortie (33) ou est disposé approximativement en son centre et la trajectoire d'accélération (S) s'étend entre la buse de sortie et l'extrémité arrière du boîtier.

6. Dispositif selon la revendication 3 ou 4, **caractérisé en ce que** l'entraînement magnétique (16) comprend une bobine magnétique prévue sur le poussoir d'éjection (20) lui-même ainsi qu'un cylindre de fer et/ou une bobine de stator entourant le poussoir d'éjection (20).

7. Dispositif selon l'une des revendications 3 à 6, **caractérisé en ce que** le poussoir d'éjection (20) est muni d'un dispositif d'accumulation de courant, afin d'alimentation l'entraînement magnétique (16) en courant.

8. Dispositif selon la revendication 1, **caractérisé en ce que** les ouvertures de compensation de pression sont reliées entre elles par l'intermédiaire d'une conduite de décharge (37).

9. Dispositif selon l'une des revendications précédentes, **caractérisé en ce que** le dispositif d'éjection (18) comprend des moyens pour la production d'une augmentation de pression dans le réservoir de liquide (24) après l'application de l'impulsion.

10. Dispositif selon la revendication 9, **caractérisé en ce que** les moyens de production d'une augmentation de pression sont constitués globalement du poussoir d'éjection (20) qui, après l'application de l'impulsion, agit, au moyen d'un entraînement (16) exerçant une force, sur le réservoir de liquide.

11. Dispositif selon la revendication 10, **caractérisé en ce que** l'entraînement exerçant une force (16) est un entraînement électromagnétique (16) pour le poussoir d'éjection (20).

12. Dispositif selon l'une des revendications précédentes, **caractérisé en ce que** le réservoir de liquide est logé dans un récipient de liquide (24) qui peut être disposé de préférence de manière interchangeable dans le boîtier (14).

13. Dispositif selon l'une des revendications précédentes, **caractérisé en ce que** le récipient de liquide (24) avec le liquide qui y est contenu est logé ou peut être logé conjointement avec le poussoir d'éjection (20) dans le boîtier (14) ou la trajectoire d'accélération (S) qui est prévue à l'intérieur et **en ce que** le boîtier (14) comprend, au niveau de son extrémité de sortie avant, une butée (30) pour le récipient de liquide (24).

14. Dispositif selon la revendication 26, **caractérisé en ce que** la butée (30) et/ou le récipient de liquide (24) sont munis d'un amortisseur de butée (29), par exemple un élément tampon en élastomère.

15. Dispositif selon l'une des revendications précédentes, dans lequel un récipient de liquide est disposé, qui comprend au moins une chambre à piston contenant un premier liquide à injecter dans un substrat, qui comprend une sortie de liquide et un élément d'application d'impact pour l'application d'une impulsion pouvant être appliquée sur le récipient de liquide.
